# EUROPÄISCHE PATENTSCHRIFT

(11) **EP 2 400 915 B1**
(45) Veröffentlichungstag und Bekanntmachung des Hinweises auf die Patenterteilung: **28.08.2013**
(21) Anmeldenummer: 10706598.9
(22) Anmeldetag: 24.02.2010
(51) Int. Cl.: A61C 1/08

(54) **BOHRSCHABLONE UND VERFAHREN ZU DEREN HERSTELLUNG**
DRILL TEMPLATE AND METHOD OF ITS MANUFACTURE
GABARIT DE PERÇAGE ET PROCÉDÉ DE SA FABRICATION

(30) Priorität: 27.02.2009 DE 102009010699
(43) Veröffentlichungstag der Anmeldung: 04.01.2012
(73) Patentinhaber: Abboud, Marcus, 53721 Siegburg (DE)
(72) Erfinder: Abboud, Marcus, 53721 Siegburg (DE)
(74) Vertreter: Eberlein, Jasper
(86) Internationale Anmeldenummer: PCT/EP2010/052333
(87) Internationale Veröffentlichungsnummer: WO 2010/097405

(56) Entgegenhaltungen:
- EP-A1- 2 072 018
- WO-A1-2008/038471
- WO-A1-2008/117323
- WO-A1-2010/018565

## Beschreibung

Die Erfindung betrifft ein Verfahren zur Herstellung einer Bohrschablone zum Bohren eines Bohrlochs in einem Patlenten-Kiefer für das Einsetzen eines Zahn-Implantats.

Als Träger für einen Zahnersatz, beispielsweise für eine Zahnkrone oder eine Zahnbrücke, kann ein im Folgenden Zahn-Implantat genanntes Implantat verwendet werden. Hierbei handelt es sich in der Regel um ein rotationssymmetrisches Implantat, und hierbei in der Regel um ein Schraubenimplantat. Bevor das rotationssymmetrische Implantat in den Patienten-Kiefer eingefügt wird, wird der Patienten-Kiefer durch Bohren eines Implantat-Bohrloches präpariert.

Die räumliche Lage des Implantat-Bohrloches im Patienten-Kiefer und die auf dem Implantat schließlich zu befestigende Zahnkrone werden an einem Computer und mit Hilfe eines zuvor bildgebend erfassten dreidimensionalen digitalen Kiefer-Modells geplant, das den Patienten-Kiefer und die vorhandenen Patienten-Zähne zeigt. Nachdem das Implantat-Bohrioch im Kiefer geplant und räumlich festgelegt ist, muss dieses genau so, wie es geplant wurde, in den Patienten-Kiefer gebohrt werden.

Hierzu wird eine Bohrschablone verwendet, die auf den Patienten-Kiefer beziehungsweise, soweit vorhanden, auf die Patienten-Zähne in einer definierten räumlichen Lage aufgesetzt wird und zur Führung des Bohrers einen Bohrkanal aufweist, der auch als Führungskanal bezeichnet werden kann. Damit die Bohrschablone in einer definierten räumlichen Lage zum Patlenten-Kiefer aufgesetzt werden kann, muss sie ein entsprechendes Negativ der Zahn-Okklusalflächen beziehungsweise des Patienten-Kiefers aufweisen. Der Bohrschablonen-Bohrkanal dient schließlich für den Bohrer, mit dem die Implantat-Bohrung in den Patlenten-Kiefer gebohrt wird, als axiale Führung und kann gegebenenfalls auch als Tiefenanschlag dienen.

In DE 199 52 962 A ist beschrieben, wie aus den gesamten digitalen dreidimensionalen Informationen des Patienten-Kiefers, der Patienten-Zähne etc. digital das Zahn-Implantat und die korrespondierende Implantat-Bohrung geplant wird. Hieraus wird schließlich eine komplexe Bohrschablone mit dem Negativ der Okklusal-Flächen der noch vorhandenen Patienten-Zähne und mit einem Bohrschablonen-Bohrkanal gefertigt. Auch aus EP 1 043 960 ist es bekannt, eine komplette Bohrschablone aus einem Stück herzustellen.

Die Herstellung derartiger Bohrschablonen ist insbesondere wegen der komplexen Gestalt des Negativs der Okklusalflächen der Nachbarzähne des geplanten Implantates technisch anspruchsvoll, so dass hierfür eine teure CAM-Maschine erforderlich ist und relativ lange Werstellungszeiten bei der Nerstellung der Bohrschablone anfallen.

Aus DE 195 10 294 A ist eine verstellbare Bohrvorrichtung bekannt, mit deren Hilfe in einer Operationsschablone ein Bohrkanal gebohrt wird, so dass schließlich eine Bohrschablone zum Bohren des für das geplante Implantat notwendigen Bohrlochs in den Patlenten-Kiefer zur Verfügung steht.

Aus WO 2008/117323 A1 ist ein Verfahren bekannt, bei dem zum Bohren einer Implantatbohrung in den Patientenkiefer ein System bestehend aus einer Prothese und einem Bohrmodell eingesetzt wird. Die Prothese besteht aus einem an den Patienten-Kiefer angepassten Teil und weist ferner eine Platte mit Referenzmitteln in Form von Löchern auf. Die Platte besteht komplett aus radiologisch sichtbarem Material. Die Löcher dienen als Kupplungsteile. Nach der radiologischen Erfassung der Prothese einschließlich der Platte wird ein Bohrmodell Individuell hergestellt.

Aufgabe der Erfindung ist es demgegenüber, ein Verfahren zur Herstellung einer Bohrschablone und eine Bohrschablone hierfür zu schaffen, das bzw. die wenig Aufwand zur Herstellung der fertigen Bohrschablone erfordert.

Diese Aufgabe wird erfindungsgemäß gelöst mit dem Verfahren zur Herstellung einer Bohrschablone mit den Merkmalen gemäß Patentanspruch 1 bzw. mit einer Bohrschablone mit den Merkmalen des Patentanspruches 5.

Zunächst wird erfindungsgemäß eine Prothese an den Kiefer und/oder die Zähne des Patienten angepasst. Diese Prothese wird im Folgenden auch als Referenzplatte bezeichnet, da sie bei der Erfassung eines dreidimensionalen digitalen Kiefer-Modells, beim Halten des Bohrmodells und schließlich als Bohrschablone als räumliche Referenz dient, die einen definierten festen räumlichen Bezug zu dem Patienten-Kiefer beziehungsweise zu den Patienten-Zähnen herstellt.

Die Referenzplatte (Prothese) kann beispielsweise dadurch hergestellt werden, dass mit Hilfe einer Abdruckmasse ein Negativ-Abdruck von dem Patienten-Kiefer beziehungsweise von den Patienten-Zähnen genommen wird. Dieser Negativ-Abdruck ist Teil der Referenzplatte, so dass die Referenzplatte (Prothese) stets in identischer räumlicher Position auf den Patienten-Kiefer beziehungsweise auf die Patienten-Zähne aufgesetzt werden kann. Ferner weist die Referenzplatte (Prothese) mindestens ein Kupplungsteil und mindestens zwei Referenzmarker auf. Die Referenzmarker sind für alle folgenden bildgebenden Verfahren opak, das heißt, insbesondere sichtbar bei der folgenden Erfassung eines dreidimensionalen digitalen Kiefer-Modells.

Nach dem Aufsetzen der Referenzplatte (Prothese) auf den Patienten-Kiefer beziehungsweise auf die Patienten-Zähne wird durch bekannte bildgebende Verfahren und Vorrichtungen ein dreidimensionales digitales Kiefer-Modell des Kiefers und der Zähne des Patienten sowie der Referenzmarker der aufgesetzten Referenzplatte (Prothese) gemacht.

Nach dem Erfassen des Kiefer-Modells wird an einem Computer das Zahn-Implantat digital geplant und konstruiert. Insbesondere wird die räumliche Lage des Zahn-Implantats in dem Patienten-Kiefer geplant und festgelegt, womit auch die räumliche Lage und Tiefe des Bohrlochs in dem Patienten-Kiefer festgelegt wird, in das schließlich das Implantat eingesetzt beziehungsweise eingeschraubt werden soll. Durch die Festlegung der räumlichen Lage des Bohrlochs in dem Patienten-Kiefer wird auch die räumliche Lage eines Bohrkanals in der Bohrschablone festgelegt.

Anschließend erfolgt - vorzugsweise automatisiert - das digitale Konstruieren eines Bohrmodell, das im Folgenden auch als Transferschablone bezeichnet wird, anhand des digitalen dreidimensionalen Kiefer-Modells und des digital konstruierten Implantats einschließlich des geplanten und räumlich festgelegten Kiefer-Bohrlochs. Die auf diese Weise konstruierte Transferschablone (Bohrmodell) ist an die Referenzplatte (Prothese) und insbesondere an die Kupplungsteile der Referenzplatte (Prothese) derart angepasst, dass die Transferschablone (Bohrmodell) in einer definierten Lage und Position an der Referenzplatte (Prothese) fixiert werden kann. Die konstruierte Transferschablone (Bohrmodell) weist insbesondere einen Bohrmodell-Bohrkanal auf, der exakt mit dem späteren Referenzplatten-/Bohrschablonen-Bohrkanal und mit dem geplanten Kiefer-Bohrloch für das Implantat axial ausgerichtet ist, wenn die Transferschablone auf der Referenzplatte und diese auf dem Kiefer fixiert ist.

Auf Grundlage des das geplante Zahn-Implantat und insbesondere das geplante Implantat-Bohrloch enthaltenden digitalen Kiefer-Modells wird die Transferschablone (Bohrmodell) hergestellt, wobei die Transferschablone (Bohrmodell) einen Führungs-Bohrkanal und ein Kupplungsteil aufweist, das auf das Kupplungsteil der Referenzplatte (Prothese) so abgestimmt ist, dass die Transferschablone (Bohrmodell) in einer definierten Lage und Position an der Referenzplatte (Prothese) fixiert werden kann.

Die Transferschablone (Bohrmodell) wird mit der Referenzplatte (Prothese) derart verbunden, dass die Transferschablone (Bohrmodell) durch die Kupplungsteile in einer definierten Lage und Position an der Referenzplatte (Prothese) fixiert ist.

Schließlich wird mit einem Bohrer, der in dem Transferschablonen-Bohrkanal geführt ist, in die Referenzplatte (Prothese) ein Referenzplatten-Bohrkanal gebohrt, wodurch die Referenzplatte (Prothese) zu einer Bohrschablone gemacht wird.

Die Bohrschablone wird von dem Zahnarzt auf den Patienten-Kiefer beziehungsweise auf die Patienten-Zähne in der durch den Negativ-Abdruck definierten Position aufgesetzt. Der Bohrkanal der Bohrschablone dient beim anschließenden Bohren des Bohrlochs in den Patienten-Kiefer als Führung für den Bohrer.

Das erfindungsgemäße Verfahren ermöglicht es, auf einfache Weise eine Bohrschablone herzustellen. Dadurch, dass die Transferplatte (Prothese) schließlich die Bohrschablone bildet, wird eine besonders genaue Positionierung der Bohrschablone am Patienten-Kiefer ermöglicht, da die Bohrschablone unmittelbar und ohne Zwischenformen aus der Transferplatte (Prothese) konstruiert wird.

Die in dem erfindungsgemäßen Verfahren verwendete Transferplatte (Prothese) kann dabei vorzugsweise einen standardisierten Teil aufweisen, der die Kupplungsteile und die Referenzmarker umfasst sowie einen individualisierten Teil, der an das Körperteil, also an den Patienten-Kiefer und/oder die Patienten-Zähne, angepasst wird beziehungsweise ist. Die Transferschablone (Bohrmodell) kann ebenfalls ein standardisiertes Teil sein, das an den standardisierten Teil der Referenzplatte (Prothese) angepasst ist. In die standardisierte Transferschablone (Bohrmodell) wird beispielsweise durch eine digital gesteuerte Bohrmaschine der Bohrkanal gebohrt.

In der Praxis kann mit dem standardisierten Transferschablonen und Referenzplatten wie folgt verfahren werden:

Zunächst nimmt der behandelnde Zahnarzt von dem betroffenen Patienten-Kiefer mit Hilfe einer geeigneten Abdruckmasse einen (negativen) mechanischen Abdruck des betroffenen Patienten-Kiefers beziehungsweise der zugeordneten Patienten-Zähne. Der Zahnarzt verfügt über einen Vorrat standardisierter Referenzplatten-Plattenteile.

Nach dem Aushärten der Abdruckmasse wird der so gewonnene Negativ-Abdruckkörper mit seiner der Abdruckseite gegenüberliegenden ebenen Rückseite auf einer Referenzplatte fixiert, beispielsweise aufgeklebt. Der Abdruckkörper bildet ein individualisiertes Teil und wird mit dem standardisierten Plattenteil zu einer Referenzplatte zusammengeführt.

Alternativ kann die Abdruckmasse auch schon vor der Abdrucknahme bereits auf das Referenzplatten-Plattenteil appliziert werden, und die Abdruckmasse zusammen mit dem Plattenteil zur Abdrucknahme auf dem Patienten-Kiefer aufgebracht werden. Zum Aushärten der Abdruckmasse kann die Abdruckmasse zusammen mit den Referenzplatten-Plattenteil wieder von dem Kiefer entfernt werden. Selbstverständlich kann die Abdrucknahme auch extra-oral an einem so genannten Realmodell des Patienten-Kiefers erfolgen.

Nach dem Aushärten der Abdruckmasse wird die Referenzplatte mit der Abdruck-Seite wieder auf den Patienten-Kiefer aufgesetzt. Anschließend wird mit bekannten zweidimensionalen und dreidimensionalen bildgebenden Verfahren ein dreidimensionales digitales Kiefer-Modell einschließlich des Patienten-Kiefers, der Zähne und der Referenzmarker der Referenzplatte angefertigt.

An einem Computer plant und konstruiert der Zahnarzt anschließend das Zahn-Implantat in dem digitalen Kiefer-Modell. Hierbei wird insbesondere die räumliche Lage der Implantat-Längsachse im Patienten-Kiefer festgelegt, und konkret die räumliche Lage und gegebenenfalls Tiefe des Implantat-Bohrlochs im Patienten-Kiefer bestimmt. Der gesamte Datensatz, bestehend aus dem digitalen Kiefer-Modell und den Informationen über die räumliche Lage des Implantat-Bohrlochs, kann nun von dem Zahnarzt an einen zentralen Transferschablonen- Herstellungsort beispielsweise über das Internet übermittelt werden.

An dem Transferschablonen- Herstellungsort wird aus dem übermittelten Kiefer-Modell-Datensatz ein Bohrkanal in die standardisierte Transferschablone gebohrt, und zwar derart, dass dieser Bohrkanal mit dem geplanten Implantat-Bohrloch genau fluchtet, wenn die Transferschablone auf der auf den Patienten-Kiefer aufgesetzten Referenzplatte fixiert wäre. Nach dem Bohren des Bohrkanals wird die Transferschablone an den Zahnarzt versendet.

Der Zahnarzt setzt die Transferschablone in einer durch die Kupplungsteile definierten und standardisierten Position auf die Referenzplatte auf, steckt einen Bohrer in den Transferschablonen-Bohrkanal, und bohrt in Ausrichtung mit dem Transferschablonen-Bohrkanal einen Bohrkanal in die Referenzplatte. Anschließend wird die Transferschablone von der Referenzplatte wieder entfernt. Die nunmehr den Bohrkanal aufweisende Referenzplatte ist damit zu einer Bohrschablone geworden, die auf den Patienten-Kiefer aufgesetzt werden kann.

Nach dem Aufsetzen auf den Patienten-Kiefer dient der Bohrkanal der Bohrschablone dem Zahnarzt als Führungsbohrung für den Bohrer, mit dem das Bohrloch für das Implantat in den Patienten-Kiefer gebohrt wird.

Es kann vorgesehen sein, dass die Referenzplatte (Prothese) und/oder die Transferschablone (Bohrmodell) einen Anlenkpunkt zum Anlenken einer Bissgabel aufweisen.

Auch ist es möglich, dass die Referenzplatte (Prothese) und/oder die Transferschablone (Bohrmodell) mit einem Artikulator verbindbar sind.

Auf diese Art und Weise ist es möglich, die in der Prothese und/oder dem Bohrmodell enthaltenen Informationen mit Hilfe einer Simulation der Kiefergelenksbewegungen zu überprüfen oder für die Konstruktion eines Zahnersatzes zu verwenden.

Vorzugsweise ist vorgesehen, dass das erfindungsgemäße Verfahren folgenden weiteren Schritt aufweist:
Digitales Konstruieren eines Zahnersatzes anhand des digitalen dreidimensionalen Kiefer-Modells oder des digital konstruierten Implantats, wobei der Zahnersatz an das digitale Oberflächenmodell angepasst ist.

Die Erfindung ermöglicht somit in vorteilhafter Weise, dass anhand der gleichen digitalen Modelle, die für die Konstruktion des Implantats verwendet wurden, auch der später an dem Implantat befestigte Zahnersatz konstruiert werden kann. Dadurch ist eine besonders genaue Konstruktion des Zahnersatzes möglich. Durch das Anpassen des Zahnersatzes an das digitale Oberflächenmodell ist eine Anpassung des Zahnersatzes an die benachbarten Zähne, die sogenannten Pfeilerzähne, in vorteilhafter Art und Weise möglich.

In einem weiteren Schritt kann vorgesehen sein, dass der Zahnersatz hergestellt wird, vorzugsweise mit einem CAM-Verfahren.

Die Erfindung sieht ferner eine Bohrschablone zum Präparieren eines Knochens für ein medizinisches Implantat bzw. zum Bohren eines Bohrlochs für ein Zahn-Implantat vor, die eine Referenzplatte (Prothese) aufweist, die an zumindest einen Teilbereich eines Körperteils bzw. den Patienten-Kiefer angepasst ist, wobei die Transferplatte (Prothese) Kupplungsteile zum Ankuppeln von Modellteilen, insbesondere eines Bohrmodells, aufweist und sieht ferner ein Bohrmodell vor, das an das medizinische Implantat und die Prothese angepasst ist, wobei die Prothese und das Bohrmodell über die Kupplungsteile miteinander verbindbar sind. Die Bohrschablone ist hier als System zu verstehen, das aus der Prothese und dem Bohrmodell gebildet wird.

Insbesondere in Verbindung mit dem erfindungsgemäßen Verfahren wird somit eine kostengünstig herstellbare Bohrschablone bereit gestellt, die in vorteilhafter Welse sehr genau auf einem Teilbereich eines Körperteils beziehungsweise auf dem Kiefer positioniert werden kann.

Es ist vorgesehen, dass die Referenzplatte (Prothese) mindestens zwei Referenzmarker aufweist, die für alle folgenden bildgebenden Verfahren opak und zum Bilden von Referenzpunkten in digitalen Bilden der Referenzplatte (Prothese) geeignet sind. Auf diese Weise wird ermöglicht, dass eine digitale Konstruktion des Bohrmodells mit Hilfe des beschriebenen Verfahrens ermöglicht wird.

Die Prothese ist berorzugt zweiteilig ausgeführt, und weist ein an dem zumindest einen Teilbereich des Körperteils beziehungsweise an den Kiefer angepasstes individualisiertes Teil und ein standardisiertes Plattenteil auf.

Auf diese Weise ist eine kostengünstige Herstellung der Prothese in vorteilhafter Welse möglich,

Dabei ist vorgesehen, dass das standardisiertes Plattenteil die mindestens zwei Referenzmarker und die Kupplungsteile aufweist.

Es kann vorgesehen sein, dass das Bohrmodell und/oder die Prothese einen Anlenkpunkt zum Anlenken einer Bissgabel aufweisen.

Ferner kann vorgesehen sein, dass die Prothese und/oder das Bohrmodell mit einem Artikulator verbindbar sind.

Auf diese Weise kann die in der Prothese und/oder dem Bohrmodell enthaltene Information bei einer Simulation der Kiefergelenksbewegung überprüft werden oder für die Konstruktion eines Zahnersatzes verwendet werden.

Ein Verfahren zur Planung eines medizinischen Implantats kann die folgenden Schritte aufweisen:
- Herstellen einer Prothese, die an zumindest einen Teilbereich eines Körperteils angepasst ist, wobei die Prothese mindestens zwei Referenzmarker aufweist,
- Erstellen von mindestens einer transversalen Schichtaufnahme des zumindest einen Teilbereichs des Körperteils, wobei während der Erstellung der Aufnahmen die Prothese auf dem Körperteil aufgesetzt ist, derart, dass die mindestens zwei Referenzmarker der Prothese in mindestens einer der transversalen Schichtaufnahmen erkennbar sind,
- Segmentieren der mindestens einen transversalen Schichtaufnahme und Erstellen eines digitalen dreidimensionalen Modells von zumindest einem Teil des zumindest einem Teilbereichs des Körperteils aus der mindestens einen transversalen Schichtaufnahme, wobei das digitale dreidimensionale Modell Positionsdaten der mindestens zwei Referenzmarker enthält,
- Ermitteln von Oberflächendaten des zumindest einen Teilbereichs des Körperteils oder eines Realmodells des zumindest einen Teilbereichs des Körperteils, wobei während der Ermittlung der Oberflächendaten die Prothese auf dem Körperteil oder auf dem Realmodell aufgesetzt ist und Erstellen eines digitalen dreidimensionalen Oberflächenmodells des zumindest einen Teilbereichs des Körperteils mit den Oberflächendaten, wobei das digitale dreidimensionale Oberflächenmodell Positionsdaten der mindestens zwei Referenzmarker enthält,
- Zusammenführen des digitalen Oberflächenmodells und des digitalen dreidimensionalen Modells und Positionieren des digitalen dreidimensionalen Modells in Bezug zu dem digitalen Oberflächenmodell mit Hilfe der Positionsdaten der mindestens zwei Referenzmarker,
- digitales Konstruieren des medizinischen Implantats an dem digitalen dreidimensionalen Modell sowie dessen Position in dem digitalen dreidimensionalen Modell, wobei das digital konstruierte Implantat und dessen Position an das digitale Oberflächenmodell angepasst ist.

Das Verfahren hat den Vorteil, dass nur für einen Teil des mindestens einen Teilbereichs des Körperteils dreidimensionale Daten der innenliegenden Bereiche vorliegen müssen, wobei diese Daten aus zweidimensionalen Schichtaufnahmen erstellt werden. Da die Prothese an dem zumindest einen Teilbereich des Körperteils angepasst ist, kann die Prothese jederzeit exakt an der gleichen Stelle des Körperteils positioniert werden, so dass bei der Erstellung der Schichtaufnahmen und bei der Ermittlung der Oberflächendaten die Prothese in Bezug zu dem Körperteil stets exakt die gleiche Position aufweist.

Dadurch ist ein genaues Positionieren des aus den Schichtaufnahmen erstellten dreidimensionalen Modells in Bezug auf das aus den Oberflächendaten erstellten digitalen dreidimensionalen Oberflächenmodell mit Hilfe der Referenzmarker, die beispielsweise in Form von Kugeln an der Prothese angeordnet sind, möglich.

Zur Ermittlung der Oberflächendaten ist es nicht zwangsläufig notwendig, dass ein Oberflächen-Scan des zumindest einen Teilbereichs des Körperteils aufgenommen wird, sondern es ist auch möglich, ein bereits vorhandenes Realmodell des zumindest einen Teilbereichs des Körperteils mit aufgesetzter Prothese zu scannen. Beispielsweise bei Verwendung des Verfahrens zur Planung von zahnmedizinischen Implantaten kann dies von Vorteil sein, da häufig von Patienten bereits Realmodelle eines Teilbereichs des Körperteils, wie beispielsweise eines Teilbereichs des Kiefers, vorliegen. Dadurch muss der Patient keine weitere Ermittlung von Daten über sich ergehen lassen, denn die Oberflächendaten können mit Hilfe des Realmodells und der Prothese im Labor ermittelt werden. Das Realmodell kann dabei in herkömmlicher Weise mit einem Abdruck erstellt worden sein. Auch ist es möglich, dass dem Realmodell ermittelte medizinische Daten, wie beispielsweise Daten eines Oberflächenscans einer Computertomographie oder einer digitalen Volumentomographie (DVT), zugrunde liegen.

Da in dem digitalen dreidimensionalen Modell auch innenliegende Gewebeteile des zumindest einen Teilbereichs des Körperteils erfasst sind, ist die Konstruktion eines medizinischen Implantats in vorteilhafter Weise möglich, da beispielsweise in dem Körperbereich liegende Nervenbahnen oder ähnliches berücksichtigt werden können. Gleichzeitig ist durch die Positionierung des digitalen dreidimensionalen Modells in Bezug auf das digitale dreidimensionale Oberflächenmodell eine Anpassung des digital konstruierten Implantats an die Umgebung möglich. Beispielsweise ist es bei der Verwendung des Verfahrens im zahnmedizinischen Bereich möglich, die Implantate an benachbarte Zähne anzupassen, da die Lage und Größe der benachbarten Zähnen dem Oberflächenmodell entnehmbar sind.

Es kann vorgesehen werden, dass zusätzlich zu der mindestens einen transversalen Schichtaufnahme eine Panoramaschichtaufnahme oder eine Längsschnittaufnahme des zumindest einen Teilbereichs des Körperteils erstellt wird, wobei während der Erstellung der Panoramaschichtaufnahme oder der Längsschnittaufnahme die Prothese auf dem Körperteil aufgesetzt ist, derart, dass mindestens einer der mindestens zwei Referenzmarker der Prothese auf der Panoramaschichtaufnahme oder der Längsschnittaufnahme erkennbar ist und dass für die Erstellung des digitalen dreidimensionalen Modells zusätzlich der mindestens einen transversalen Schichtaufnahme die Panoramaschichtaufnahme oder die Längsschnittaufnahme verwendet wird.

Da mit Röntgenapparaten, mit denen transversale Schichtaufnahmen möglich sind, zumeist auch Panoramaschichtaufnahmen oder Längsschnittaufnahmen erstellbar sind, kann eine derartige Panoramaschichtaufnahme oder Längsschnittaufnahme ohne großen Aufwand und Kosten erstellt werden. Da die Panoramaschichtaufnahme oder Längsschnittaufnahme in etwa orthogonal zu mindestens einer der in etwa parallel verlaufenden transversalen Schichtaufnahmen verläuft, kann durch die Hinzunahme der Panoramaschichtaufnahme oder der Längsschnittaufnahme die Genauigkeit des digitalen dreidimensionalen Modells verbessert werden. Selbstverständlich kann dabei vorgesehen sein, dass die Panoramaschichtaufnahme oder die Längsschnittaufnahme bei der Erstellung des digitalen dreidimensionalen Modells segmentiert wird.

In dem Verfahren kann ein weiterer Schritt vorgesehen sein, der das Herstellen eines Realmodells betrifft, das zumindest einen Teilbereich eines Körperteils zeigt, wobei vorzugsweise die Prothese mit Hilfe des Realmodells hergestellt wird und/oder die Oberflächendaten anhand des Realmodells ermittelt werden. Die Herstellung des Realmodells kann beispielsweise als erster Schritt des Verfahrens durchgeführt werden.

Durch die Herstellung eines Realmodells ist die Herstellung der Prothese sowie die Ermittlung der Oberflächendaten auf eine einfache Art und Weise im Labor möglich, so dass kein zusätzlicher Arztbesuch des Patienten notwendig ist. Für die Herstellung des Realmodells können ein herkömmlicher Abdruck oder die Daten eines Oberflächen- Scans des zumindest einen Teilbereichs des Körperteils verwendet werden. Zusätzlich oder alternativ können auch Daten einer Computertomographie oder einer digitalen Volumentomographie verwendet werden.

Im Folgenden wird unter Bezugnahme auf die nachfolgenden Zeichnungen die Erfindung näher erläutert. Es zeigen:
- Fig. 1: den Ablauf eines Verfahrens,
- Fig. 2: eine schematische Darstellung eines Unterkiefers mit aufgesetzter erfindungsgemäßer Prothese und
- Fig. 3: eine schematische Ansicht eines Unterkiefers mit aufgesetzter Prothese und Bohrmodell.

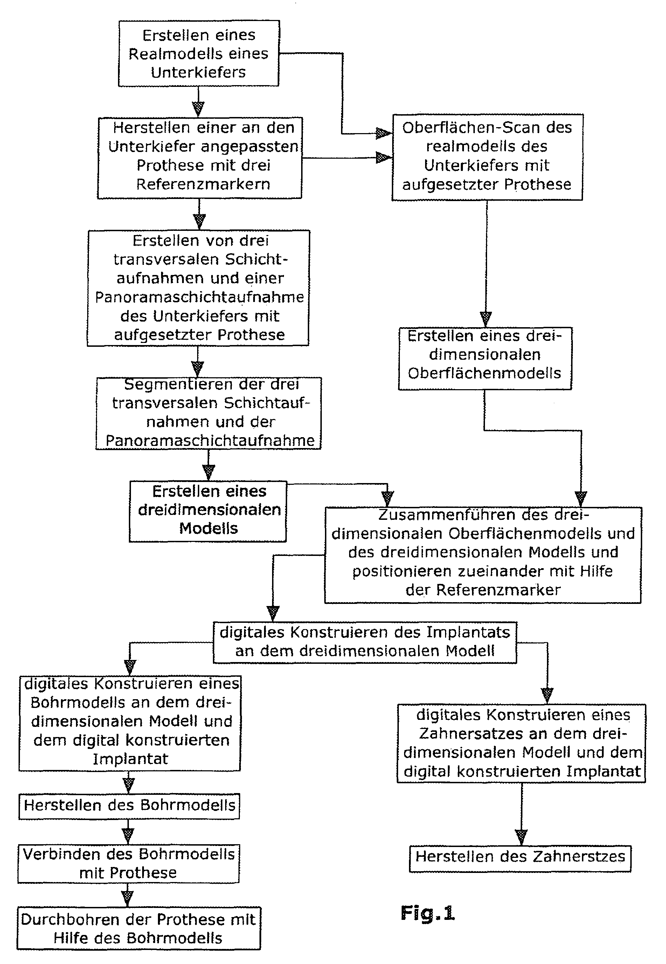

In Fig. 1 ist der Ablauf eines Verfahrens zur Planung eines zahnmedizinischen Implantats am Unterkiefer eines Patienten schematisch dargestellt.

Zunächst wird ein Realmodell des Unterkiefers 1 und der Zähne 5 des Patienten auf herkömmliche Art und Weise hergestellt. Dies kann durch einen Oberflächenscan des Kiefers 1 und der Zähne 5 oder auch anhand von Computertomographie oder digitaler Volumentomographiedaten geschehen. Selbstverständlich ist es auch möglich, das Realmodell in herkömmlicher Weise mit Hilfe einer mechanischen Abdrucknahme des Kiefers 1 zu erstellen.

Anschließend wird eine an den Unterkiefer 1 angepasste Prothese 3 mit zwei oder drei Referenzmarkern 7 hergestellt. Die Prothese 3 kann an das Realmodell des Unterkiefers 1 oder direkt an den Unterkiefer 1 des Patienten angepasst werden. Die Anpassung der Prothese 3 an den Kiefer 1 und/oder die Zähne 5 kann beispielsweise konventionell durch eine Abdrucknahme mit bekannten Abdruckmassen erfolgen. Der ausgehärtete Kiefer-Abdruck 3b, der ein individuelles Teil 3b der Prothese 3 darstellt, kann mit einem standardisierten Prothesen-Plattenteil 3a zu der Prothese 3 verbunden, beispielsweise verklebt, werden.

An dem Realmodell des Unterkiefers 1 mit aufgesetzter Prothese 3 wird ein Oberflächen-Scan erstellt, wobei der Oberflächen-Scan insbesondere auch Positionsdaten der Referenzmarker 7 der Prothese 3 aufnimmt. Alternativ ist es selbstverständlich möglich, den Oberflächen-Scan auch an dem Unterkiefer 1 des Patienten zu erstellen, wobei hierbei die Prothese 3 auf dem Unterkiefer 1 des Patienten aufgesetzt ist.

Aus dem Oberflächen-Scan des Unterkiefers 1 mit aufgesetzter Prothese 3 wird ein dreidimensionales Oberflächenmodell erstellt, wobei die Positionsdaten der Referenzmarker 7 der Prothese 3 in dem Oberflächenmodell enthalten sind.

Ferner werden drei transversale Schichtaufnahmen und eine Panoramaschichtaufnahme oder eine Längsschnittaufnahme des Unterkiefers 1 des Patienten erstellt, wobei während der Aufnahmen die Prothese 3 auf dem Unterkiefer 1 des Patienten aufgesetzt ist. Die transversalen Schichtaufnahmen und die Panoramaschichtaufnahme bzw. die Längsschnittaufnahme werden von dem Bereich des Unterkiefers 1 des Patienten erstellt, in dem später das Implantat eingesetzt werden soll. Die Längsschnittaufnahme verläuft dabei orthogonal zu zumindest einer der transversalen Schichtaufnahmen und vorzugsweise mittig zu zumindest einer der transversalen Schichtaufnahmen.

Die transversale Schichtaufnahme und die Panoramaschichtaufnahme bzw. die Längsschnittaufnahme können mit einem herkömmlichen dentalen Röntgengerät erstellt werden.

Beim nächsten Schritt werden die transversalen Schichtaufnahmen und die Panoramaschichtaufnahme bzw. die Längsschnittaufnahme segmentiert, indem digital die Bereiche der Aufnahmen, die zu einem Gewebeteil des Unterkiefers bzw. Zahns gehören, auf den unterschiedlichen Aufnahmen markiert werden. Die auf den Aufnahmen ebenfalls erkennbaren Referenzmarker 7 der Prothese 3 werden ebenfalls segmentiert.

Mit Hilfe der Segmentierung der einzelnen Bereiche der Aufnahmen kann ein dreidimensionales Modell des aufgenommenen Bereichs des Unterkiefers 1 digital erstellt werden, wobei in dem dreidimensionalen Modell die Positionsdaten der Referenzmarker 7 der aufgesetzten Prothese 3 enthalten sind.

Das digital erstellte dreidimensionale Modell kann anschließend mit dem aus dem Oberflächen- Scan erstellten dreidimensionalen Oberflächenmodell zusammengeführt werden, wobei die beiden Modelle mit Hilfe der Referenzmarker 7 exakt zueinander positioniert werden können.

Durch das Zusammenführen der beiden Modelle wird ein gemeinsames Kiefer-Modell erstellt, in dem der Bereich des Unterkiefers 1, in den das Implantat eingesetzt werden soll, in dreidimensionaler Form mit Informationen über die innenliegenden Gewebebereiche vorliegt, während die übrigen Bereiche des Unterkiefers nur als Oberflächendaten vorliegen.

Anhand dieses Kiefer-Modells kann nun in dem dreidimensionalen Modell das Implantat digital konstruiert werden. Dabei können nicht nur die in dem Bereich des Unterkiefers 1, in den das Implantat eingesetzt werden soll, vorhandenen innenliegenden Gewebeteile berücksichtigt werden, sondern das Implantat kann auch an die benachbarten Zähne 5, die aus dem dreidimensionalen Oberflächenmodell ersichtlich sind, angepasst werden.

Anhand der digitalen Konstruktion des Implantats kann nun ein passendes Implantat ausgesucht bzw. hergestellt werden.

Mit Hilfe der erstellten digitalen Kiefer-Modelle wird eine Bohrschablone 10 erstellt, die für die Bohrung des für das Implantat notwendigen Bohrlochs 21 in dem Kiefer 1 verwendet wird. Dabei kann vorgesehen sein, dass anhand des dreidimensionalen Modells und des digital konstruierten Implantats ein Bohrmodell 11 digital konstruiert wird.

Anschließend kann das Bohrmodell 11 hergestellt werden, beispielsweise mit Hilfe eines 3D-Druckers oder mit Hilfe eines aufbauenden Verfahrens. Insbesondere ist vorgesehen, dass das Bohrmodell 11 an die Prothese 3 angepasst ist. Daher ist es auch möglich, dass das Bohrmodell 11 ein standardisiertes Teil ist, wobei die durch die digitale Konstruktion erstellte Form des Bohrmodells 11 in herkömmlicher Weise durch ein abtragendes Verfahren auf das standardisierte Teil übertragen wird. Das fertige Bohrmodell 11 weist einen Bohrkanal 15 auf, der eine Bohrführung zum Bohren des Bohrkanals 20 in der Prothese 3 ist.

Anschließend wird das Bohrmodell 11 mit der Prothese 3 verbunden. Zu diesem Zweck kann die Prothese 3 beispielsweise Verbindungsteile und/oder Kupplungsteile 9 aufweisen, an die das Bohrmodell 11 bzw. an die Bohrmodell-Kupplungsteile 13 angepasst ist.

Anschließend wird mit Hilfe des Bohrmodells 11 in der Prothese 3 der Prothesen-Bohrkanal 20 gebohrt, so dass die Prothese 3 in Alleinstellung oder in Verbindung mit dem Bohrmodell 11 die Bohrschablone 10 bilden kann.

Die Verwendung der Prothese 3 als Bohrschablone 10 bzw. als Teil der Bohrschablone 10 ist deshalb von Vorteil, weil mit Hilfe der Prothese 3 sowohl die für die Konstruktion der Bohrschablone 10 notwendigen Daten gewonnen werden als auch das Positionieren der Bohrschablone 10 für das Bohren des Bohrlochs 21 mit Hilfe derselben Prothese 3 erfolgt. Dadurch ist ein sehr exaktes Positionieren der Bohrschablone 10 auf dem Kiefer 1 zum Bohren 11 Bohrlochs 21 in dem Kiefer 1 sichergestellt.

Das Verfahren kann ferner vorsehen, dass anhand des dreidimensionalen Modells und des digital konstruierten Implantats ein Zahnersatz digital konstruiert werden kann, wobei der Zahnersatz an das dreidimensionale Oberflächenmodell angepasst sein kann. Somit ist es möglich, anhand der gleichen Daten, die für die Konstruktion des Implantats verwendet werden, auch den Zahnersatz zu konstruieren, wobei der Zahnersatz in vorteilhafter Weise an das digital konstruierte Implantat und an die benachbarten Zähne, die sich aus dem dreidimensionalen Oberflächenmodell ergeben, angepasst werden kann.

In einem weiteren Schritt kann dann der Zahnersatz hergestellt werden, beispielsweise mit Hilfe eines CAM-Verfahrens, das ein aufbauendes Verfahren, wie z.B. Stereolythographie, oder 3D-Druck oder ein abtragendes Verfahren, wie z.B. ein Fräsverfahren, verwenden kann.

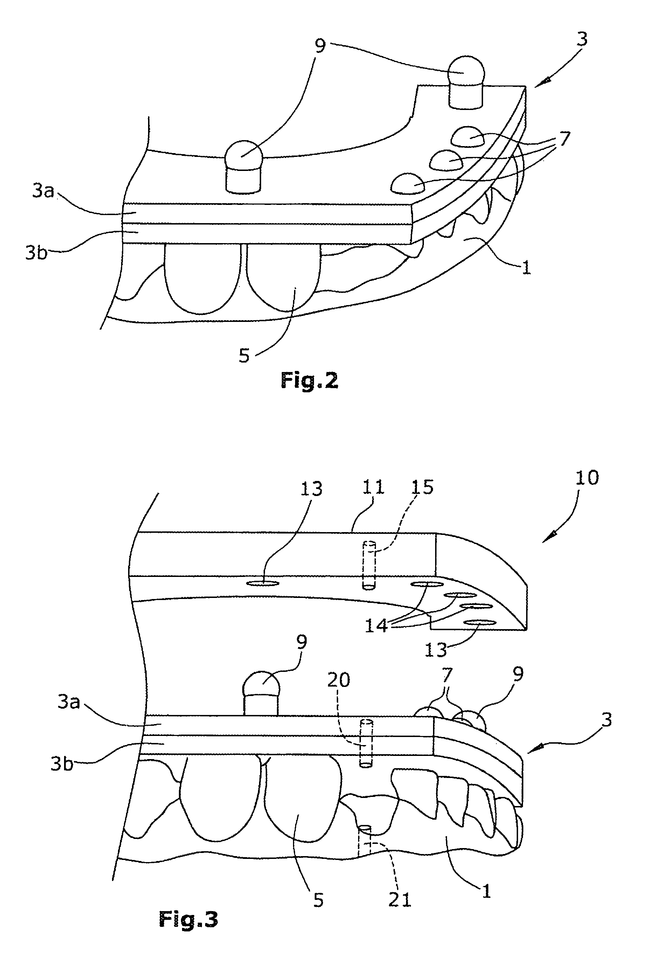

In Fig. 2 ist exemplarisch ein Unterkiefer 1 eines Patienten mit aufgesetzter Prothese 3 schematisch dargestellt.

In dem in Fig. 2 und 3 dargestellten Ausführungsbeispiel überdeckt die Prothese 3 jeweils alle noch vorhandenen Zähne 5 des Unterkiefers 1 des Patienten. Selbstverständlich ist es auch möglich, dass die Prothese 3 nur an einen Teilbereich des Unterkiefers 1 angepasst ist und nur einen Teilbereich des Unterkiefers 1 überdeckt.

Die Prothese 3 weist eine an die Zähne 5 des Unterkiefers 1 angepasste Unterseite (Vorderseite) auf. An der Oberseite (Rückseite) der Prothese 3 sind zwei Referenzmarker 7 schematisch dargestellt. Die Referenzmarker 7 sind in dem in den Fig. 2 und 3 dargestellten Ausführungsbeispiel jeweils als metallische Halbkugeln ausgebildet. Selbstverständlich sind auch andere Formen der Referenzmarker möglich, die auf den im Rahmen des oben beschriebenen Verfahrens aufgenommenen Schichtaufnahmen sowie in dem in dem oben beschriebenen Verfahren erstellten Oberflächen-Scan erkennbar sind.

Ferner weist die Prothese 3 mehrere Kupplungsteile 9 auf, die zum Ankuppeln von Modellteilen, insbesondere zum Ankuppeln des Bohrmodells 11 geeignet sind.

Die Prothese 3 kann einteilig ausgeführt sein. Vorzugsweise ist die Prothese 3 mehrteilig ausgeführt ist, wobei vorgesehen ist, dass die Prothese 3 ein standardisiertes Plattenteil 3a aufweist, an oder in dem die Referenzmarker 7 sowie die Kupplungsteile 9 angeordnet sind, und ein individualisiertes Teil 3b umfasst, das an den Kiefer 1 des Patienten angepasst ist, beispielsweise durch Abdrucknahme mit bekannten Abdruckmassen.

In Fig. 3 ist eine erfindungsgemäße Bohrschablone 10 schematisch dargestellt. Die Bohrschablone 10 besteht aus der Prothese 3 sowie einem Bohrmodell 11. Das Bohrmodell 11 ist an die Prothese 3 angepasst und lässt sich mit der Prothese 3 verbinden. Dazu weist das Bohrmodell 11 an der Unterseite Kupplungs-Öffnungen 13 auf, die an die Kupplungsteile 9 der Prothese 3 angepasst sind und daher bohrmodellseitige Kupplungsteile 13 bilden. Ferner sind an der Unterseite des Bohrmodells 11 Vertiefungen 14 vorgesehen, die an die Referenzmarker 7 angepasst sind. Auf diese Weise ist es möglich, das Bohrmodell 11 bündig mit der Prothese 3 zu verbinden.

Das Bohrmodell 11 weist einen Bohrkanal 15 auf, der mit Hilfe des erfindungsgemäßen Verfahrens konstruiert und auf das Bohrmodell 11 übertragen worden ist. Dabei kann das Bohrmodell 11 ein standardisiertes Teil sein, auf das der Bohrkanal 15 mit Hilfe eines auftragenden oder abtragenden Verfahrens übertragen worden ist, beispielsweise durch Bohren des Bohrkanals 15. Das gesamte Bohrmodell 11 kann alternativ entsprechend der Konstruktion mit dem oben beschriebenen Verfahren als Individualteil hergestellt werden.

Mit Hilfe des Bohrmodells 11, das in einer durch die Kupplungsteile 9,13 definierten Position auf die Prothese 3 aufgesetzt wird, kann nun ein eine Bohrführung bildender Bohrkanal 20 in der Prothese 3 erstellt beziehungsweise gebohrt werden. Die Prothese 3 kann in Alleinstellung oder zusammen mit dem Bohrmodell 11 die Bohrschablone 10 für das Bohren eines Bohrlochs 21 in dem Unterkiefer 1 bilden, in das anschließend das Implantat eingesetzt, beispielsweise eingeschraubt, werden kann.

Da in dem beschriebenen Verfahren sowohl für die Konstruktion des Implantats, des Bohrlochs 21 für das Implantat in dem Kiefer 1 und der Bohrschablone 10 dieselbe Prothese 3 verwendet wird, und dieselbe Prothese 3 schließlich auch für die Positionierung der Bohrschablone 10 zum Einsatz kommt, ist ein sehr genaues Präparieren des Kiefers 1 beziehungsweise Bohren des Bohrlochs 21 für das Implantat in den Kiefer 1 möglich.

In einem nicht dargestellten Ausführungsbeispiel können die Prothese und/oder das Bohrmodell Anlenkpunkte für einen Bissbogen und/oder einen Artikulator aufweisen, so dass das Bohrmodell bzw. die Prothese auch bei der Simulation der Kiefergelenksbewegung eingesetzt werden können. Dadurch ist es beispielsweise möglich, die Prothese und/oder das Bohrmodell zu überprüfen oder die Prothese und/oder das Bohrmodell können bei der Anfertigung oder Überprüfung von Zahnersatz verwendet werden.

## Patentansprüche

1. Verfahren zur Herstellung einer Bohrschablone (10) zum Bohren eines Bohrlochs (21) in einem Patienten-Kiefer (1) für das Einsetzen eines Zahnimplantats, mit den Verfahrensschritten:
• Anfertigen und Anpassen einer Prothese (3) an den Kiefer (1) und/oder die Zähne (5) des Patienten, wobei die Prothese (3) mindestens ein Kupplungsteil (9) und mindestens zwei Referenzmarker (7) aufweist, die bei der folgenden Erfassung eines dreidimensionalen digitalen Kiefermodells opak sind.
• Aufsetzen der Prothese (3) auf den Kiefer (1) und/oder die Zähne (5),
• Erfassen eines dreidimensionalen digitalen Kiefer-Modells einschließlich des Patienten-Kiefers (1), der Zähne (5) und der Referenzmarker (7),
• Planen und digitales Konstruieren eines Zahn-Implantats in dem digitalen Kiefer-Modell,
• Herstellen eines Bohrmodells (11), das einen Bohrmodell-Bohrkanal (15) aufweist, mit Hilfe des das geplante Zahn-Implantat zeigenden digitalen Kiefer-Modells, wobei das Bohrmodell (11) ein Kupplungsteil (13) aufweist, das auf das Kupplungsteil (9) der Prothese (3) abgestimmt ist,
• Montage des Bohrmodells (11) auf der Prothese (3) mit Hilfe der Kupplungsteile (9,13), und
• Bohren eines Prothesen-Bohrkanals (20) in die Prothese (3) in Ausrichtung mit dem Bohrmodell-Bohrkanal (15), wobei die Prothese (3) mit dem Bohrkanal (20) die Bohrschablone (10) zum Bohren des für das Implantat notwendigen Bohrlochs (21) in dem Patienten-Kiefer bildet.

2. Verfahren zur Herstellung einer Bohrschablone (10) nach Anspruch 1, wobei die Prothese (3) zweiteilig ausgeführt ist und ein an die Zähne (5) angepasstes individualisiertes Teil (3b) und ein standardisiertes Plattenteil (3a) aufweist.

3. Verfahren zur Herstellung einer Bohrschablone (10) nach Anspruch 2, wobei das standardisierte Plattenteil (3a) die mindestens zwei Referenzmarker (7) und das Kupplungsteil (9) aufweist.

4. Verfahren zur Herstellung einer Bohrschablone (10) nach einem der Ansprüche 1 bis 3, wobei das Bohrmodell (11) ein standardisiertes Teil ist, in das der Bohrkanal (15) gebohrt wird.

5. Bohrschablone (10) zum Bohren eines Bohrlochs (21) in einem Patienten-Kiefer für ein medizinisches Zahnimplantat, mit einer Prothese (3), die an die Zähne (5) des Patienten-Kiefers angepasst ist, wobei die Prothese (3) Kupplungsteile (9) zum Ankuppeln eines Bohrmodells (11) und mindestens zwei Referenzmarker (7) aufweist, die für alle folgenden bildgebenden Verfahren opak sind und zum Bilden von Referenzpunkten in digitalen Bildern der Prothese geeignet sind, wobei das Bohrmodell (11) einen an das Zahnimplantat angepassten Bohrkanal (15) und ein Kupplungsteil (13) aufweist, und derart an die Prothese (3) angepasst ist, dass die Prothese (3) und das Bohrmodell (11) über ihre Kupplungsteile (9, 13) miteinander verbindbar sind.

6. Bohrschablone (10) nach Anspruch 5, wobei die Prothese (3) zweiteilig ausgeführt ist und ein an die Zähne (5) angepasstes, individualislertes Teil (3b) und ein standardisiertes Plattenteil (3a) aufweist.

7. Bohrschablone (10) nach Anspruch 6, wobei das standardisierte Plattenteil (3a) die mindestens zwei Referenzmarker (7) und das Kupplungsteil (9) aufweist.

8. Bohrschablone nach einem der Ansprüche 5 bis 7, wobei das Bohrmodell (11) ein standardisiertes Teil ist, in das der Bohrkanal (15) gebohrt wird.

## Claims

1. A method for the production of a drill template (10) for drilling a drill hole (21) in a patient's jaw (1) for inserting a tooth implant therein, comprising the following steps:
• producing and adapting a prosthesis (3) to the jaw (1) and/or the teeth (5) of the patient, the prosthesis (3) comprising at least one coupling part (9) and at least two reference markers (7) that are opaque during the subsequent capture of a three-dimensional digital jaw model,
• placing the prosthesis (3) on the jaw (1) and/or the teeth (5),
• capturing a three-dimensional digital jaw model including the patient jaw (1), the teeth (5) and the reference markers (7),
• planning and digitally constructing a tooth implant in the digital jaw model,
• forming a drilling model (11) comprising a drilling model drill channel (15), using the digital jaw model showing the planned tooth implant, wherein the drilling model (11) comprises a coupling part (13) adapted to the coupling part (9) of the prosthesis (3),
• mounting the drilling model (11) on the prosthesis (3) using the coupling parts (9, 13), and
• drilling a prosthesis drill channel (20) into the prosthesis (3) in alignment with the drilling model drill channel (15), wherein the prosthesis (3) with the drill channel (20) forms the drill template (10) for drilling the drill hole (21) in the patient's jaw that is necessary for the implant.

2. The method for the production of a drill template (10) of claim 1, wherein the prosthesis (3) is of a bipartite design and comprises an individualized part (3b) adapted to the teeth (5) and a standardized plate part (3a).

3. The method for the production of a drill template (10) of claim 2, wherein the standardized plate part (3a) comprises said at least two reference markers (7) and the coupling part (9).

4. The method for the production of a drill template (10) of one of claims 1 to 3, wherein the drilling model (11) is a standardized part into which the drill channel (15) is drilled.

5. A drill template (10) for drilling a drill hole (21) into a patient's jaw for a medical tooth implant, comprising a prosthesis (3) adapted to the teeth (5) of the patient's jaw, wherein the prosthesis (3) comprises coupling parts (9) for coupling a drilling model (11) thereto and at least two reference markers (7) that are opaque to all subsequent imaging processes and are suited to form reference points in digital images of the prosthesis (3), wherein the drilling model (11) comprises a drill channel (15) adapted to the tooth implant and a coupling part (13) and is adapted to the prosthesis (3) such that the prosthesis (3) and the drilling model (11) can be connected to each other through their coupling parts (9, 13).

6. The drill template (10) of claim 5, wherein the prosthesis (3) is of bipartite design and comprises an individualized part (3b) adapted to the teeth (5) and a standardized plate part (3a).

7. The drill template (10) of claim 6, wherein the standardized plate part (3a) comprises the at least two reference markers (7) and the coupling part (9).

8. The drill template of one of claims 5 to 7, wherein the drilling model (11) is a standardized part into which the drill channel (15) is drilled.

## Revendications

1. Procédé de fabrication d'un gabarit de perçage (10) pour le perçage d'un trou de perçage (21) dans une mâchoire d'un patient (1) destiné pour l'insertion d'un implant dentaire, avec les étapes de procédé suivantes:
• fabriquer et adapter une prothèse (3) à la mâchoire (1) et/ou les dents (5) du patient, ladite prothèse (3) comprenant au moins un élément de couplage (9) et au moins deux marqueur de référence (7) qui sont opaques lors de la capture suivante d'un modèle de mâchoire numérique tridimensionnelle,
• placer la prothèse (3) sur la mâchoire et /ou les dents (5),
• capturer un modèle de mâchoire numérique tridimensionnelle, incluant la mâchoire du patient (1) les dents (5) et les marqueurs de référence (7),
• concevoir et construire numériquement un implant dentaire dans ledit modèle de mâchoire numérique,
• fabriquer un modèle de perçage (11) avec un canal de perçage (15) dans le modèle de perçage, à l'aide dudit modèle de mâchoire numérique présentant l'implant dentaire conçu, ledit modèle de perçage (11) comprenant un élément de couplage (13) adapté à l'élément de couplage (9) de la prothèse (3),
• monter ledit modèle de perçage (11) sur la prothèse (3) à l'aide des éléments de couplage (9, 13), et
• percer un canal de perçage de prothèse (20) dans ladite prothèse (3) en alignement avec ledit canal de perçage (15) dans le modèle de perçage, ladite prothèse (3) avec ledit canal de perçage (20) forme le gabarit de perçage (10) pour le perçage du trou de perçage (21) dans la mâchoire du patient, nécessaire pour l'implant.

2. Procédé de fabrication d'un gabarit de perçage (10) selon la revendication 1, dans lequel la prothèse (3) est conçue en deux parties et comprend une partie individualisée (3b), adaptée aux dents (5), et une partie de plaque standardisée (3a).

3. Procédé de fabrication d'un gabarit de perçage (10) selon la revendication 2, dans lequel la partie de plaque standardisée (3a) comprend les au moins deux marqueurs de référence (7) et ledit élément de couplage (9).

4. Procédé de fabrication d'un gabarit de perçage (10) selon l'une des revendications 1 à 3, dans lequel ledit modèle de perçage (11) est une partie standardisée dans laquelle est percé ledit canal de perçage (15).

5. Gabarit de perçage (10) pour le perçage d'un trou de perçage (21) dans une mâchoire d'un patient destiné à un implant dentaire médical, comprenant une prothèse (3) adaptée aux dents (5) de ladite mâchoire d'un patient, ladite prothèse (3) comprenant des éléments de couplage (9), pour y accoupler un modèle de perçage (11), et au moins deux marqueurs de référence (7) qui sont opaques pour tout les procédés d'imagerie suivants et sont aptes à former des points de référence dan des images numériques de ladite prothèse, ledit modèle de perçage (11) comprenant un canal de perçage (15), adapté à l'implant dentaire, et un élément de couplage (13) et étant adapté à ladite prothèse (3) de sorte que ladite prothèse (3) et le modèle de perçage (11) peuvent être connectés l'un à l'autre par l'intermédiaire de leurs éléments de couplage (9, 13).

6. Gabarit de perçage (10) selon la revendication 5, dans lequel ladite prothèse (3) est conçue en deux parties et comprend une partie individualisée (3b), adaptée aux dents (5), et une partie de plaque standardisée (3a).

7. Gabarit de perçage (10) selon la revendication 6, dans lequel la partie de plaque standardisée (3a) comprend les au moins deux marqueurs de référence (7) et ledit élément de couplage (9).

8. Gabarit de perçage (10) selon l'une des revendications 5 à 7, dans lequel ledit modèle de perçage (11) est une partie standardisée dans laquelle est percé ledit canal de perçage (15).
